# DEMANDE DE BREVET EUROPEEN

(11) **EP 2 664 919 A1**
(43) Date de publication de la demande: **20.11.2013**
(21) Numéro de dépôt: 12168121.7
(22) Date de dépôt: 15.05.2012
(51) Int. Cl.: G01N 33/50, G01N 33/68

(54) **Une méthode pour identifier les ligands du récepteur AhR possédant une activité sebosuppressive thérapeutique**

(71) Demandeur: Saurat, Jean Hilaire, 1206 Geneve (CH)
(72) Inventeur: Saurat, Jean Hilaire, 1206 Geneve (CH)
(74) Mandataire: Bugnion Genève

(57) **Abrégé**

Méthode de tri de substances en vue de déterminer leur aptitude à une activité sebosuppressive dans le cadre d'un traitement topique de la peau, comprenant un test in vivo, le dit test in vivo comprenant les étapes suivantes :
- choisir une substance parmi les ligands du récepteur AhR,
- choisir un mammifère exprimant le gène CYP1A1,
- traiter par voie topique une partie de la peau du dit mammifère comprenant des glandes sébacées avec une composition comprenant la dite substance, selon un protocole dose/réponse(s)/temps de réponse,
- examiner par marquage immunohistochimique l'expression de CYP1A1 dans des glandes sébacées de ladite partie de la peau du dit mammifère,
- sélectionner ladite substance en fonction de la séquence d'apparition du marquage immunohistochimique dans plusieurs types différents de cellules desdites glandes sébacées.

## Description

### INTRODUCTION

La présente invention concerne une méthode de tri de substances en vue de déterminer leur aptitude à une activité sebosuppressive dans le cadre d'un traitement topique de la peau.

De nombreuses substances sont susceptibles d'interagir en tant que ligands avec le récepteur AhR (Aryl hydrocarbon Receptor), exprimé notamment par les cellules épithéliales et mésenchymales de la peau. Les documents WO 2004/041758, WO 2007/060256 et WO 2007/128725 décrivent des tests in vitro visant à déterminer le caractère antagoniste ou agoniste de tels ligands et proposent l'utilisation de ligands antagonistes dans le cadre de traitements dermatologiques.

Le déposant a au contraire montré dans le document WO 2009/093207 que l'application sur la peau de certains ligands agonistes du récepteur AhR entrainait une forte diminution de l'activité des glandes sébacées. Le déposant a montré que cette propriété peut être utilisée pour traiter les états comportant un excès de production de sebum tels que la peau grasse (séborrhée), l'acné, etc... et comment ces ligands devaient être choisis pour qu'ils n'induisent pas une des principales complications de l'activation extravagante de AhR par les ligands xéno-toxiques tels que la dioxine, c'est à dire des lésions kystiques prolifératives. Ces lésions kystiques étaient appelées anciennement « chloracné », et dorénavant MADISH (J. H. Saurat et coll The cutaneous lesions of dioxin exposure: Lessons from the poisoning of V. Yushchenko Toxicological Sciences 2012 ; 125 : 310-317). Pour éviter que l'application de ces ligands à visée thérapeutique induisent des MADISH, il convient de les choisir en ce qu'ils soient rapidement métabolisés (demi-vie en heures/jours), contrairement à la dioxine et ses congénères dont la demi-vie se compte en années. Les ligands possédant de telles caractéristiques, tels que ceux listés dans le document WO 2009/093207, et dont la liste est incorporée ici par référence, sont nombreux et appartiennent surtout aux classes des « ligands naturels » de AhR dits NAhRA (Natural AhR Agonists).

Il est cependant difficile d'identifier, parmi ces ligands, les meilleurs candidats susceptibles d'être doués d'un effet sébosuppresseur chez l'homme. En effet ceci implique des tests d'induction de l'atrophie des zones différenciées des glandes sébacées, dans des espèces animales appropriées. Ces tests sont d'interprétation complexe et pour être sensibles peuvent nécessiter l'application prolongée sur plusieurs semaines des ligands étudiés.

D'autre part, pour l'optimalisation de l'effet thérapeutique il est important de pouvoir disposer d'une méthode sensible et rapide de tri de molécules de synthèse construites à partir des concepts que le déposant a identifiés pour les molécules naturelles.

Le but de l'invention est donc de proposer une méthode de tri telle que définie d'entrée offrant une corrélation élevée entre le résultat du tri et les propriétés sebosuppressives sur la peau humaine des substances sélectionnées.

### OBJET DE L'INVENTION

À cet effet, l'invention propose une méthode de tri de substances en vue de déterminer leur aptitude à une activité sebosuppressive dans le cadre d'un traitement topique de la peau, comprenant un test in vivo, le dit test in vivo comprenant les étapes suivantes :
- choisir une substance parmi les ligands du récepteur AhR,
- choisir un mammifère exprimant le gène CYP1A1,
- traiter par voie topique une partie de la peau du dit mammifère choisie en fonction des glandes sébacées qu'elle contient avec une composition comprenant la dite substance, selon un protocole dose/réponse(s)/temps de réponse,
- examiner par marquage immunohistochimique l'expression de CYP1A1 dans les glandes sébacées de ladite partie de la peau du dit mammifère,
- sélectionner ladite substance en fonction de la séquence d'apparition du marquage immunohistochimique dans plusieurs types (compartiments) différents de cellules desdites glandes sébacées.

On peut en particulier choisir la peau des oreilles dont on sait qu'elle contient des glandes sébacées bien analysables et dans lesquelles le gène CYP1A1 est particulièrement susceptible d'être induit.

Ladite substance est sélectionnée si le dit test in vivo fait apparaître un marquage dans une pluralité de types de cellules dans un intervalle de temps déterminé.

Ladite substance est choisie de préférence parmi les ligands du récepteur AhR présentant un caractère agoniste sur la base d'au moins un test in vitro. Le choix du meilleur candidat naturel parmi les NAhRA passe donc dans un premier temps par la démonstration d'un effet agoniste AhR suffisant, par exemple par les tests CALUX et EROD, ainsi que d'une demi-vie in vivo courte.

Selon un mode d'exécution de la méthode selon l'invention, le dit mammifère est une souris de souche C57/B6.

Selon ce mode d'execution, plus particulièrement les oreilles desdites souris sont traitées par voie topique, puis prélevées et l'expression de CYP1A1 est étudiée par immunohistochimie au moyen d'un anticorps ; en particulier mais de façon non exclusive, l'anticorps utilisé peut être l'anticorps polyclonal de lapin anti CYP1A1 de rat Millipore AB1247).

Dans ce mode d'exécution, l'examen de l'expression de CYP1A1 dans les glandes sébacées peut comprendre
- l'examen de la zone de l'isthme, en particulier l'examen des cellules progénitrices,
- de l'examen de la zone périphérique de la glande, en particulier l'examen des cellules indifférenciées,
- l'examen de la zone intermédiaire de la glande, en particulier l'examen des cellules différenciées,
- l'examen de la zone centrale de la glande, en particulier l'examen des cellules matures.

Après examen de ces quatre types cellulaires, ladite substance peut être sélectionnée si l'expression de CYP1A1 est marquée dans au moins deux types de cellules après une semaine de traitement.

Après examen de ces quatre types cellulaires ladite substance peut être sélectionnée si l'expression de CYP1A1 est marquée dans les 4 types de cellules après une semaine de traitement.

L'homme de l'art comprendra grâce à la description détaillée ci-dessous d'un mode d'exécution particulier de l'invention, en relation avec les figures accompagnantes comment il a été possible d'établir une nouvelle méthode pour identifier les ligands du recepteur AhR possédant une activité sébosuppressive thérapeutique topique.

### BREVE DESCRIPTION DES FIGURES

- La figure 1 comprend les parties 1A, 2A, 3A, 4A qui sont une représentation schématique de la localisation à l'intérieur d'une glande sébacée de quatre types de cellules susceptibles d'exprimer le gène CYP1A1, à savoir :
   - cellules progénitrices (1),
   - cellules indifférenciées (2),
   - cellules différenciées (3),
   - cellules matures (4).
- la figure 2 comprend les parties 1B, 2B, 3B, 4B qui sont des microphotographies d'observations correspondant aux dessins 1A, 2A, 3A, 4A; 1C montre un angle de vue et un grossissement différent de la zone 1B c'est-à-dire des cellules progénitrices.
- la figure 3 représente l'apparition de l'activité CYP1A1 en fonction du temps dans chacune des sous populations de cellules sébacées des figures 1 et 2, après application topique de plusieurs ligands du récepteur AhR,
- la figure 4 est un tableau qui montre les corrélations entre test in vitro, test in vivo, et examen clinique de l'activité sebosuppressive chez l'homme des ligands de la figure 3.

### DESCRIPTION DETAILLEE D'UN MODE D'EXECUTION

La méthode selon l'invention pour le tri des molécules ligands de AhR, à visée thérapeutique sébosuppressive chez l'homme, est basée sur une observation fortuite et inattendue faite lors de l'administration topique et systémique de plusieurs ligands AhR listés dans WO 2009/093207.

Pour que l'homme de l'art comprenne le caractère très surprenant de la présente invention, on doit rappeler les éléments suivants de l'état de la technique qui concernent la distribution dans la peau des phénomènes biologiques déclenchés par l'application d'un ligand pour un récepteur nucléaire tel que l'AhR, ou encore à titre d'exemple, les récepteurs pour l'acide rétinoïque (RAR), les récepteurs de la vitamine D (VDR).

Selon l'état de la technique, l'activité biologique déclenchée par l'apport par voie topique ou systémique du ligand devrait se manifester de façon uniforme :
- sur toutes les partie des l'organe qui expriment le récepteur, et
- dans lesquelles le ligand diffuse de façon quantitativement suffisante et sous forme active non métabolisée.

En l'occurrence l'application d'un ligand AhR sur la peau devrait activer la voie de ce récepteur de façon uniforme d'abord dans les couches superficielles de l'épiderme, puis progressivement en suivant le gradient de pénétration et la diffusion du ligand dans les couches profondes de l'épiderme, puis éventuellement le derme et les annexes, poils et glandes sébacées. En effet il est connu, et le déposant a confirmé ce fait, que le récepteur AhR est uniformément exprimé dans tous ces compartiments de la peau.

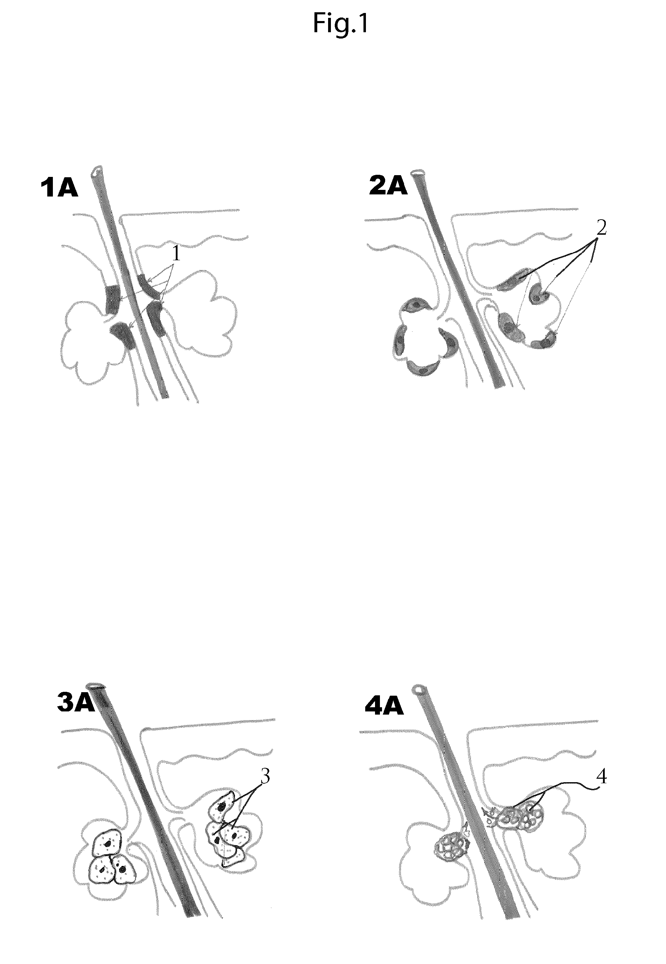

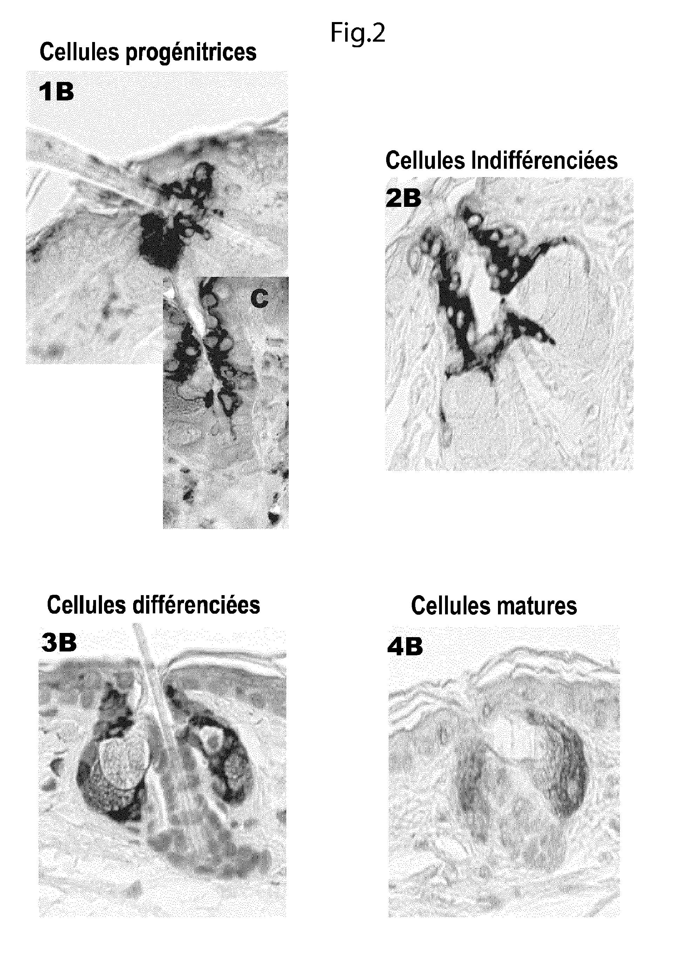

Or, le déposant a constaté que de manière très surprenante, l'application sur la peau d'un ligand AhR active la voie de ce récepteur de *façon focale sur l'appareil sébacé,* en commençant par les cellules progénitrices, c'est-à-dire les cellules souches sébacées situées dans la région isthmique du follicule pilosébacé, puis séquentiellement, les cellules sébacées non différenciées, les cellules sébacées différenciées, puis les cellules matures. Ces observations sont illustrées par les figures 1 et 2.

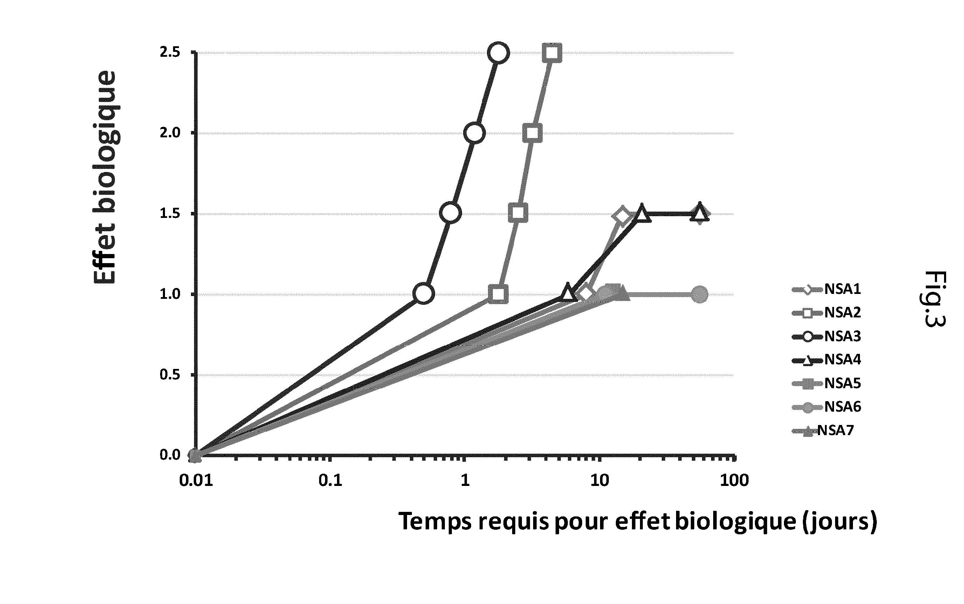

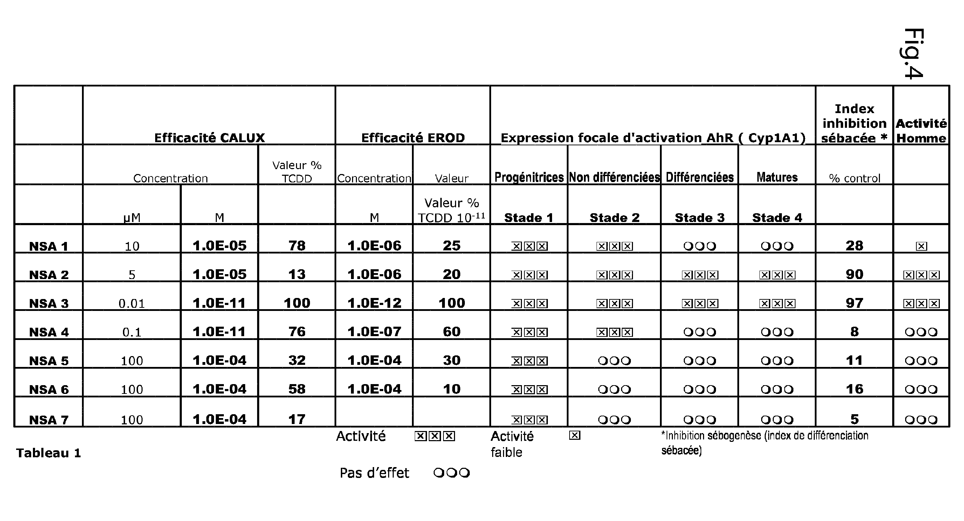

De façon encore plus surprenante, les ligands doués d'une forte activité sébosuppressive sont ceux qui, sans qu'ils soient forcement les plus actifs in vitro sur les tests d'activation du récepteurs AhR, sont ceux donc qui suivent cette séquence d'activation in situ de façon précoce, rapide et complète, comme le montre le tableau de la figure 4 ainsi que la figure 3.

Ces observations sont en effet surprenantes car elles diffèrent de celles de Rose MJ et al. (J. Invest. Derm. 2008: 128; 1866-68*).* Ces auteurs avaient montré que, sur une souris transgénique (promoteur CYP1A1-GFP) l'activité biologique induite par un ligand d'AhR administré par voie systémique pouvait se localiser dans les glandes sébacées, mais de manière uniforme à l'intérieur de ces dernières :
- d'une part, dans les observations du déposant, la voie d'administration est transcutanée, et la distribution préférentielle est totalement inattendue pour les raisons de diffusion transépidermique expliquées ci-dessus. Dans les travaux de Rose MJ et al. (J. Invest. Derm. 2008: 128; 1866-68*)* l'administration du ligand, le 3-methyl cholantrène, se fait par voie systémique, ce qui implique un accès à la peau par voie sanguine, de sorte que la distribution d'un ligand lipophile dans la glande sébacée n'est pas surprenante ;
- d'autre part et surtout, la séquence d'activation cellules progénitrices > indifférenciées> différenciés> cellules matures qui fait la base de la présente invention n'était pas observée par Rose MJ et al. (J. Invest. Derm. 2008: 128; 1866-68*),* qui montrent une activité biologique diffuse dans la zone des glandes sébacées. Cet aspect montré par Rose et al. n'aurait permis ni d'établir ni de suggérer la méthode de tri des ligands sébosuppresseurs qui fait l'objet de cette invention.

### EXEMPLES

### Démonstration des zones focales sequentielles d'activation du recepteur AhR sur l'appareil sébacé.

Le mode d'exécution décrit ici consiste à traiter par voie topique dans des protocoles dose/réponse et temps/ réponse des oreilles de souris C57/B6 avec des ligands du récepteur AhR précédemment caractérisés pour leur propriétés d'activation in vitro du récepteur, par exemple par les méthodes CALUX et EROD, qui sont des méthodes largement utilisées dans ce domaine (voir Tableau 1).

Les oreilles sont prélevées et l'expression de CYP1A1 est étudiée par immunohistochimie avec un anticorps spécifique. La figure 1 montre les différents types de marquage observés. La positivité de l'immunohistochimie, c'est-à-dire cellules marquées en brun de la figure 2, indique que la zone exprime la protéine CYP1A1.
1. À l'état basal, la protéine n'est pas détectable.
2. Dans tous les cas de ligands AhR utilisés ayant induit une positivité de l'immunohistochimie CYP1A1 , la première zone temporellement marquée, indiquant l'augmentation de la protéine CYP1A1 induite par l'activation du récepteur AhR est la zone de l'isthme où se trouvent les cellules « progénitrices » des glandes sébacées. (Fig 1A et 1B) : stade 1. Ceci correspond à des cellules clonogéniques multipotentes, donc de façon générale aux « cellules souches sébacées ». Outre la topographie particulière dans la région isthmique, ces cellules sont caractérisées par l'expression de kératine 15, et de L-RIG1, un marqueur de cellules clonogéniques multipotentes isthmiques. En utilisant un double marquage CYP1A1 et L-RIG1 il a été possible d'affirmer que les cellules activées par le ligand d'AhR, d'origine topique, correspondent en effet à cette population à ce niveau isthmique.
3. Le stade suivant est l'extension du marquage à des cellules indifférenciées qui ne contiennent pas de lipides et sont en règle générale localisées à la périphérie de la glande sébacée (Fig 2A et 2B) :stade 2.
4. Le stade suivant est l'extension du marquage à des cellules différenciées qui contiennent des lipides et sont en règle localisées à la partie intermédiaire de la glande sébacée (Fig 3A et 3B) :stade 3.
5. Le stade suivant est l'extension du marquage à des cellules matures qui contiennent des lipides et sont en règle générale localisées à la partie centrale de la glande sébacée. (Fig 4A et 4B) : stade 4.

### Correspondance du stade d'activation séquentielle avec les propriétés sebosuppressives du ligand

La tableau de la Fig 4 indique la correspondance entre les stades d'expression focale d'activation et l'index d'inhibition sébacé, ainsi que l'effet sur la peau humaine. L'index d'inhibition sébacé est calculé en comptant le nombre de cellules matures et différenciées par rapport au nombre de cellules totales dans les glandes sébacées. Une diminution des cellules matures et différenciées indique un blocage de la sébogénèse . L'effet sur la peau humaine est déterminé par examen sébumétrique dit « casual level » (J Cosmet Dermatol. 2007 jun;6(2):113-8).

Dans la figure 3 et le tableau 1 de la figure 4, les abréviations ont la signification suivante :
NSA1 rutecarpine
NSA2 beta naphtoflavone
NSA3 TCDD
NSA4 FICZ
NSA5 Hispidine
NSA6 beta carboline
NSA7 esomeprazole.

La comparaison de la figure 3 et de la figure 4 (tableau 1) montre que les deux ligands qui activent le la voie AhR dans les quatre sous populations cellulaires, et ce dans un temps inférieur à une semaine, sont aussi ceux qui, d'après l'examen clinique, montrent la plus forte activité sebosuppressive chez l'homme. Au contraire, d'autres ligands, qui n'activent le récepteur AhR que dans une sous-population de cellules, et même si cette activation atteint un niveau important, ne présentent à l'examen clinique qu'une activité sebosuppressive médiocre.

### CONCLUSION

La méthode selon l'invention, outre les informations nouvelles concernant la physiopathologie de la glande sébacée qu'elle implique, permet de rapidement faire le tri des molécules sebosuppressives candidates à une utilisation thérapeutique.

Les ligands susceptibles d'induire un effet sebosuppresseur signifiant au plan clinique sont ceux qui dès la première semaine de traitement ont dépassé le stade 2. Les plus actifs atteignent le stade 4 dès la première semaine de traitement.

L'homme du métier comprendra aisément que la méthode selon l'invention pourrait être mise en pratique, sans sortir du cadre de l'invention, au moyen d'un autre mammifère de laboratoire que la souche de souris C57/B6, à condition de reproduire la séquence d'activation décrite.

Cette discrépance entre les données in vitro, qui représentent la puissance d'activation du récepteur, et l'effet in vivo, reflètent probablement des éléments de cinétique intra tissulaire propres à chaque molécule. La méthode qui fait l'objet de cette invention est ainsi la première jamais décrite, qui permette d'explorer le ciblage spécifique des cellules sébacées, au sein du tissu cutané.

## Revendications

1. Méthode de tri de substances en vue de déterminer leur aptitude à une activité sebosuppressive dans le cadre d'un traitement topique de la peau, **caractérisée en ce que** ladite comprend un test in vivo, le dit test in vivo comprenant les étapes suivantes :
- choisir une substance parmi les ligands du récepteur AhR,
- choisir un mammifère exprimant le gène CYP1A1,
- traiter par voie topique une partie de la peau du dit mammifère comprenant des glandes sébacées avec une composition comprenant la dite substance, selon un protocole dose/réponse(s)/temps de réponse,
- examiner par marquage immunohistochimique l'expression de CYP1A1 dans des glandes sébacées de ladite partie de la peau du dit mammifère,
- selectionner ladite substance en fonction de la séquence d'apparition du marquage immunohistochimique dans plusieurs types différents de cellules desdites glandes sébacées.

2. Méthode selon la revendication 1, **caractérisée en ce que** ladite substance est sélectionnée si le dit test in vivo fait apparaître un marquage dans une pluralité de types de cellules dans un intervalle de temps déterminé.

3. Méthode selon la revendication1 ou 2, **caractérisée en ce que** ladite substance est choisie parmi les ligands du récepteur AhR présentant un caractère agoniste sur la base d'au moins un test in vitro.

4. Méthode selon la revendication 3, caractérisée que ledit test in vitro est choisi parmi les tests CALUX, EROD, et leurs combinaisons.

5. Méthode selon l'une des revendications précédentes, **caractérisée en ce que** le dit mammifère est une souche de souris choisie **en ce que** les glandes sébacées permettent de reproduire la séquence d'activation
- cellules progénitrices,
- cellules indifférenciées,
- cellules différenciées,
- cellules matures.

6. Méthode selon l'une des revendications précédentes, **caractérisée en ce que** le dit mammifèreest une souris de souche C57/B6.

7. Méthode selon la revendication 5ou 6, **caractérisée en ce que** les oreilles desdites souris sont traitées par voie topique, puis prélevées et que l'expression de CYP1A1 est étudiée par immunohistochimie au moyen d'un anticorps.

8. Méthode selon l'une des revendications précédentes, **caractérisée en ce que** l'examen de l'expression de CYP1A1 dans les glandes sébacées comprend
- l'examen de la zone de l'isthme, en particulier l'examen des cellules progénitrices,
- de l'examen de la zone périphérale de la glande, en particulier l'examen des cellules indifférenciées,
- l'examen de la zone intermédiaire de la glande, en particulier l'examen des cellules différenciées,
- l'examen de la zone centrale de la glande, en particulier l'examen des cellules matures.

9. Méthode selon la revendication 7, **caractérisée en ce que** ladite substance est sélectionnée si l'expression de CYP1A1 est marquée dans plus que deux types de cellules après une semaine de traitement.

10. Méthode selon la revendication 7, **caractérisée en ce que** ladite substance est sélectionnée si l'expression de CYP1A1 est marquée dans 4 types de cellules après une semaine de traitement.
